# EUROPEAN PATENT APPLICATION

(11) **EP 4 226 909 A1**
(43) Date of publication of application: **16.08.2023**
(21) Application number: 21877746.4
(22) Date of filing: 08.10.2021
(51) Int. Cl.: A61K 8/29, A61K 8/02, A61Q 1/02

(54) **SOFT FOCUS FILLER, COSMETIC**

(30) Priority: 08.10.2020 JP 2020170245
(71) Applicant: Fujimi Incorporated, Kiyosu-shi, Aichi 452-8502 (JP)
(72) Inventor: IWAKUNI Mayumi, Kiyosu-shi, Aichi 452-8502 (JP); ASHITAKA Keiji, Kiyosu-shi, Aichi 452-8502 (JP); MIWA Naoya, Kiyosu-shi, Aichi 452-8502 (JP)
(74) Representative: Maiwald GmbH
(86) International application number: PCT/JP2021/037320
(87) International publication number: WO 2022/075447

(57) **Abstract**

Provided is a soft focus filler excellent in both transmittance and haze. The soft focus filler of the present invention includes a powder including plate-shaped crystal particles of titanium phosphate.

## Description

### Technical Field

The present invention relates to a soft focus filler and a cosmetic.

### Background Art

Cosmetics such as foundations and lipsticks are required to have a property called soft focus property. The soft focus property means a property of hiding skin blemishes, freckles, pores, fine wrinkles, or the like by blurring the skin surface when a cosmetic is applied onto the skin to form a cosmetic coating. Having high soft focus property means that, of the light applied to a cosmetic coating film (all irradiation light), a large part does not go straight through the cosmetic coating film but is dispersed.

To impart the soft focus property to a cosmetic coating film, a soft focus filler including an inorganic powder such as silica has been added to cosmetics.

PTL 1 discloses a cosmetic additive (soft focus filler) including alumina-silica particles. In the cosmetic additive, the alumina-silica particles satisfy all the following properties: including cubic primary particles having a side length of 0.3 to 20 um observed under a scanning electron microscope; having a refractive index of 1.48 to 1.52 determined by the immersion method; having a volume-based average particle diameter of 1 to 20 um determined by the Coulter counter method; having an oil absorption value of not less than 10 ml/100 g and less than 50 ml/100 g in accordance with JIS K5101-13-2; and having a specific surface area of 20 m²/g or less determined by the BET method.

PTL 2 discloses a cosmetic including spherical organopolysiloxane particles that impart feeling of use such as silkiness and smoothness and extensibility to the cosmetic and further exert excellent soft focus effect. The cosmetic contains, as a soft focus filler, spherical organopolysiloxane particles having a volume average particle diameter of 0.1 to 30 um, having an average refractive index of 1.44 to 1.57, and containing 90% by mole or more of organosilsesquioxane units.

PTL 3 discloses a composition particularly including a physiologically acceptable medium containing a) at least one soft focus filler and b) at least one composite pigment containing at least one non-spherical aluminum oxide, at least one metal oxide, and at least one surface treating agent. As the soft focus filler, a composite powder of talc/TiO₂/alumina/silica is exemplified.

PTL 4 discloses a cosmetic including a barium sulfate doped with a certain amount of a rare earth element as a soft focus filler.

### Citation List

### Patent Literature

PTL 1: JP 2019-172648 A
PTL 2: JP 2020-75881 A
PTL 3: JP 2018-530580 A
PTL 4: JP 2014-88351 A

### Summary of Invention

### Technical Problem

The soft focus fillers disclosed in PTLs 1 to 4 are not excellent in both transmittance and haze (scattering light/total light transmittance), unfortunately.

The present invention is intended to provide a soft focus filler excellent in both transmittance and haze.

### Solution to Problem

To solve the problems, an aspect of the present invention provides a soft focus filler including a powder including plate-shaped crystal particles of titanium phosphate.

### Advantageous Effects of Invention

According to the present invention, a soft focus filler excellent in both transmittance and haze can be provided.

### Brief Description of Drawings

FIG. 1 is a graph illustrating the relation between total light transmittances and hazes of powders in Examples;
FIG. 2 is a graph illustrating the relation between volume-based diameters D50% and total light transmittances of titanium phosphate powders prepared in Examples; and
FIG. 3 is a graph illustrating the relation between volume-based diameters D50% and hazes of titanium phosphate powders prepared in Examples.

### Description of Embodiments

Embodiments of the present invention will now be described, but the present invention is not limited to the embodiments described below. The embodiments described below include technologically preferred limitations for carrying out the present invention, but the limitations are not essential requirements of the invention.

A soft focus filler in the embodiment includes a powder including plate-shaped crystal particles of titanium phosphate.

The volume-based 50% cumulative primary particle diameter (volume-based diameter D50%) is 0.1 um or more and 7.5 um or less where the primary particle diameter is determined as the dimension of the maximum diagonal on a plane of the plate-shaped crystal by the image analysis method. The volume-based 50% cumulative thickness (volume-based thickness D50%) is 0.01 um or more and 1.00 um or less where the thickness is determined as the dimension of the side face of the plate-shaped crystal, and the aspect ratio (volume-based diameter D50% divided by volume-based thickness D50%) is 5 or more.

The soft focus filler in the embodiment includes a powder including plate-shaped crystal particles of titanium phosphate and thus is excellent in both transmittance and haze.

In the soft focus filler in the embodiment, the powder including plate-shaped crystal particles of titanium phosphate has a volume-based diameter D50% of 0.10 um or more and 7.5 um or less, a volume-based thickness D50% of 0.01 um or more and 1.00 um or less, and an aspect ratio of 5 or more, and thus the soft focus filler can satisfy both a total light transmittance of 80% or more and a haze of 55% or more. The powder has an aspect ratio of 5 or more, and thus the soft focus filler also has excellent slidability.

In the soft focus filler in the embodiment, the powder including plate-shaped crystal particles of titanium phosphate preferably has a volume-based diameter D50% of 0.1 um or more and 3.0 um or less. Accordingly, the soft focus filler can satisfy both a total light transmittance of 80% or more and a haze of 80% or more.

Crystal particles having a volume-based thickness D50% of less than 0.01 um fail to form plate-shaped particles.

The soft focus filler in the embodiment includes a powder including plate-shaped crystal particles of titanium phosphate, and the powder can be prepared, for example, by the following method.

First, an aqueous solution of titanyl sulfate and an aqueous solution of phosphoric acid are mixed at such a ratio that the phosphorus molarity [P] to the titanium molarity [Ti], [P]/[Ti], is 5 or more and 21 or less, and a liquid mixture is prepared. Next, the liquid mixture is placed in a closed container and is maintained at a temperature of 100°C or more and 160°C or less to undergo reaction for a predetermined period (for example, 5 hours or more). In other words, hydrothermal synthesis is performed. The pressure in the closed container is higher than the atmospheric pressure and is naturally determined by a pressing temperature. A slurry containing crystal particles of titanium phosphate is thus prepared.

Next, the prepared slurry is cooled, and then a solid content (crystal particles of titanium phosphate) is separated from the slurry. The resulting solid content is cleaned with water or a cleaning solution containing aqueous ammonia (ammonium hydroxide) and then is dried.

### [Cosmetic]

Examples of the cosmetic containing the soft focus filler (hereinafter called a "cosmetic composition") include makeup cosmetics such as a foundation, a face powder, a cheek rouge, and a lipstick.

The soft focus filler in an embodiment of the present invention may be subjected to a treatment such as various polymer treatments and the following treatment before use for improving cosmetic characteristics or pigment characteristics. Examples of the treatment method include fluorine treatment, silicon treatment, alkyl silane treatment, alkyl titanate treatment, metallic soap treatment, lauroyl lysine treatment, ester treatment, and amino acid treatment. In the amino acid treatment, proline, hydroxyproline, alanine, glycine, sarcosine, aspartic acid, or glutamic acid may be used.

A cosmetic containing the soft focus filler in an embodiment of the present invention may contain additional components as needed as long as the effect of the invention is not impaired. Examples of the additional components include components typically used in cosmetics, such as a solvent, an oleum, a surfactant, a moisturizer, an organic ultraviolet absorber, an antioxidant, a thickener, an aromatic, a coloring agent, a physiologically active component, and an antimicrobial agent.

The additional components may be used singly or in combination of two or more of them. The content of the additional component is not specifically limited and can be appropriately set.

The content percentage of the soft focus filler in an embodiment of the present invention contained in a cosmetic is preferably 0.1% by mass or more and 50% by mass or less relative to the whole cosmetic.

### Examples

### [Preparation of powder]

### <No. 1>

First, an aqueous solution of titanyl sulfate and an aqueous solution of phosphoric acid were mixed at such a ratio that the phosphorus molarity [P] to the titanium molarity [Ti], [P]/[Ti], was 9.0, and a liquid mixture was prepared. Next, the liquid mixed was placed in a 1.4-L autoclave and was maintained at a temperature of 110°C to undergo reaction for 5 hours.

After the reaction, the lid was removed, and a slurry in the container was cooled to room temperature and then was taken out of the container. From the slurry, a solid content was separated by filtration. The solid content was cleaned with water and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. An image under the scanning electron microscope was analyzed by using an image analysis software "Mac-View ver. 4" manufactured by Mountech, and the volume-based diameter D50% and the volume-based thickness D50% of the crystal particles included in the prepared powder were determined to be a volume-based diameter D50% of 0.29 um and a volume-based thickness D50% of 0.030 um.

From the determined volume-based thickness D50% and the determined volume-based diameter D50%, the aspect ratio of the crystal particles included in the prepared powder was calculated at 10 (0.29/0.030).

### <No. 2>

First, an aqueous solution of titanyl sulfate and an aqueous solution of phosphoric acid were mixed at such a ratio that the phosphorus molarity [P] to the titanium molarity [Ti], [P]/[Ti], was 10.7, and a liquid mixture was prepared. Next, the liquid mixture was placed in a 1.4-L autoclave and was maintained at a temperature of 110°C to undergo reaction for 5 hours.

After the reaction, the lid was removed, and a slurry in the container was cooled to room temperature and then was taken out of the container. From the slurry, a solid content was separated by filtration. The solid content was cleaned with water and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. Substantially the same procedure as in No. 1 was performed, and the volume-based diameter D50% and the volume-based thickness D50% of the crystal particles included in the prepared powder were determined to be a volume-based diameter D50% of 0.53 um and a volume-based thickness D50% of 0.065 um.

From the determined volume-based thickness D50% and the determined volume-based diameter D50%, the aspect ratio of the crystal particles included in the prepared powder was calculated at 8 (0.53/0.065).

### <No. 3>

First, an aqueous solution of titanyl sulfate and an aqueous solution of phosphoric acid were mixed at such a ratio that the phosphorus molarity [P] to the titanium molarity [Ti], [P]/[Ti], was 10.4, and a liquid mixture was prepared. Next, the liquid mixture was placed in a 1.4-L autoclave and was maintained at a temperature of 110°C to undergo reaction for 5 hours.

After the reaction, the lid was removed, and a slurry in the container was cooled to room temperature and then was taken out of the container. From the slurry, a solid content was separated by filtration. The solid content was cleaned with water and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. Substantially the same procedure as in No. 1 was performed, and the volume-based diameter D50% and the volume-based thickness D50% of the crystal particles included in the prepared powder were determined to be a volume-based diameter D50% of 0.74 um and a volume-based thickness D50% of 0.090 um.

From the determined volume-based thickness D50% and the determined volume-based diameter D50%, the aspect ratio of the crystal particles included in the prepared powder was calculated at 8 (0.74/0.090).

### <No. 4>

First, an aqueous solution of titanyl sulfate and an aqueous solution of phosphoric acid were mixed at such a ratio that the phosphorus molarity [P] to the titanium molarity [Ti], [P]/[Ti], was 10.2, and a liquid mixture was prepared. Next, the liquid mixture was placed in a 200-L autoclave and was maintained at a temperature of 110°C to undergo reaction for 5 hours.

After the reaction, the lid was removed, and a slurry in the container was cooled to room temperature and then was taken out of the container. From the slurry, a solid content was separated by filtration. The solid content was cleaned with 29% aqueous ammonia (an aqueous solution of an ammonium salt) and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. Substantially the same procedure as in No. 1 was performed, and the volume-based diameter D50% and the volume-based thickness D50% of the crystal particles included in the prepared powder were determined to be a volume-based diameter D50% of 1.11 um and a volume-based thickness D50% of 0.143 um.

From the determined volume-based thickness D50% and the determined volume-based diameter D50%, the aspect ratio of the crystal particles included in the prepared powder was calculated at 8 (1.11/0.143).

### <No. 5>

First, an aqueous solution of titanyl sulfate and an aqueous solution of phosphoric acid were mixed at such a ratio that the phosphorus molarity [P] to the titanium molarity [Ti], [P]/[Ti], was 6.9, and a liquid mixture was prepared. Next, the liquid mixture was placed in a 1.4-L autoclave and was maintained at a temperature of 120°C to undergo reaction for 5 hours.

After the reaction, the lid was removed, and a slurry in the container was cooled to room temperature and then was taken out of the container. From the slurry, a solid content was separated by filtration. The solid content was cleaned with water and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. Substantially the same procedure as in No. 1 was performed, and the volume-based diameter D50% and the volume-based thickness D50% of the crystal particles included in the prepared powder were determined to be a volume-based diameter D50% of 2.07 um and a volume-based thickness D50% of 0.302 um.

From the determined volume-based thickness D50% and the determined volume-based diameter D50%, the aspect ratio of the crystal particles included in the prepared powder was calculated at 7 (2.07/0.302).

### <No. 6>

First, an aqueous solution of titanyl sulfate and an aqueous solution of phosphoric acid were mixed at such a ratio that the phosphorus molarity [P] to the titanium molarity [Ti], [P]/[Ti], was 10.8, and a liquid mixture was prepared. Next, the liquid mixture was placed in a 200-L autoclave and was maintained at a temperature of 130°C to undergo reaction for 5 hours.

After the reaction, the lid was removed, and a slurry in the container was cooled to room temperature and then was taken out of the container. From the slurry, a solid content was separated by filtration. The solid content was cleaned with water and then was dried (by standing at a temperature of 105°C for 24 hours), and a powder was prepared.

The prepared powder was analyzed by using an X-ray diffractometer, and the result revealed that the particles included in the powder were a crystalline titanium phosphate having a structural formula of Ti(HPO₄)₂·H₂O.

The prepared powder was observed under a scanning electron microscope, and the result revealed that the particles included in the powder had a plate-shaped shape, and many hexagonal plates were included. Substantially the same procedure as in No. 1 was performed, and the volume-based diameter D50% and the volume-based thickness D50% of the crystal particles included in the prepared powder were determined to be a volume-based diameter D50% of 7.44 um and a volume-based thickness D50% of 0.856 um.

From the determined volume-based thickness D50% and the determined volume-based diameter D50%, the aspect ratio of the crystal particles included in the prepared powder was calculated at 9 (7.44/0.856).

### <No. 7>

A commercially available titanium dioxide powder having a volume-based diameter D50% of 0.29 um was prepared.

### <No. 8>

A commercially available boron nitride powder having a volume-based diameter D50% of 9.94 um was pulverized in a pot mill to prepare a boron nitride powder having a volume-based diameter D50% of 4.24 um and a volume-based thickness D50% of 0.395 um. From the determined volume-based thickness D50% and the determined volume-based diameter D50%, the aspect ratio of the crystal particles included in the powder was calculated at 11 (4.24/0.395).

### <No. 9>

A commercially available boron nitride powder having a volume-based diameter D50% of 9.75 um and a volume-based thickness D50% of 0.966 um was prepared. The aspect ratio of the crystal particles included in the powder was 10 (9.75/0.966).

### <No. 10>

A commercially available boron nitride powder having a volume-based diameter D50% of 9.94 um and a volume-based thickness D50% of 2.088 um was prepared. The aspect ratio of the crystal particles included in the powder was 5 (9.94/2.088).

### [Measurement of total light transmittance and haze]

The total light transmittance and the haze of each powder in No. 1 to No. 10 were determined by the following method.

First, each powder and an acrylate silicone coating former KP-545 (coating component: (acrylates/dimethicone) copolymer) manufactured by Shin-Etsu Chemical were weighed at a mass ratio of powder:KP-545 = 10:90 and were mixed with a homomixer, and each powder slurry was prepared. An appropriate amount of the prepared slurry was placed on a slide glass and was applied and dried so as to give a film thickness of 25 um, and a test sample of each powder was prepared. The test sample was subjected to a haze meter to determine the total light transmittance and the haze.

### [Determination of wrinkle blurring effect]

As described above, the soft focus property means a property of hiding skin blemishes, freckles, pores, fine wrinkles, or the like by blurring the skin surface when a cosmetic is applied onto the skin to form a cosmetic coating. To evaluate the soft focus property of each powder in No. 1 to No. 10, 0.005 g of each powder was placed on a black artificial leather cut out into a square with a side-length of 5 cm and was spread with a sponge. The spread powder was visually inspected by 10 persons, and the wrinkle blurring effect of each powder was evaluated.

Specifically, the 10 persons graded the wrinkle blurring effect on a scale of 1 to 5 (5: very high, 4: high, 3: somewhat high, 2: somewhat low, 1: low), and the samples were evaluated into the following five levels: a sample having a total point of 43 or more and 50 or less was evaluated as "very high"; a sample having a total point of 35 or more and 42 or less was evaluated as "high"; a sample having a total point of 27 or more and 34 or less was evaluated as "somewhat high"; a sample having a total point of 19 or more and 26 or less was evaluated as "somewhat low"; and a sample having a total point of 10 or more and 18 or less was evaluated as "low".

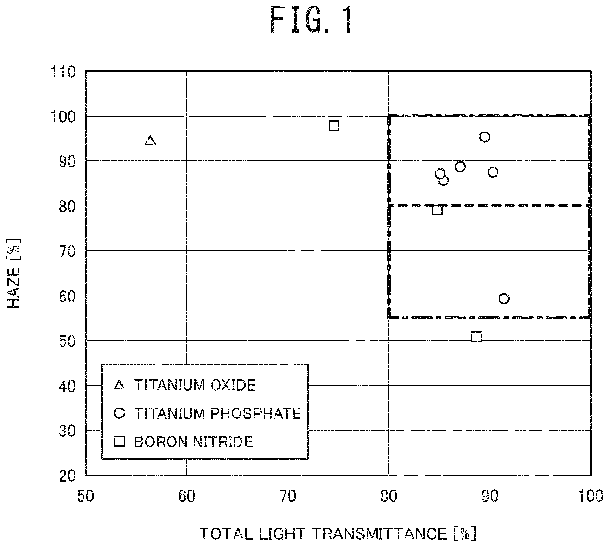

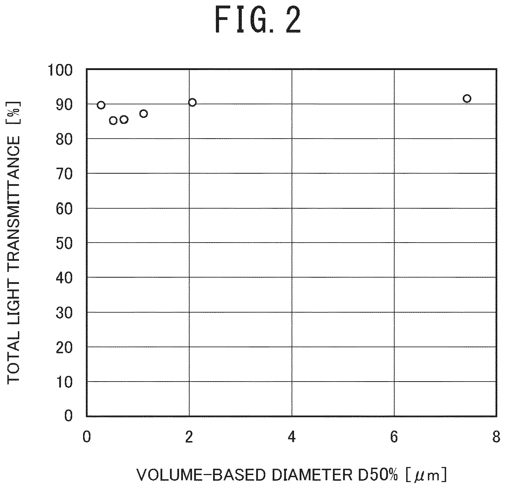

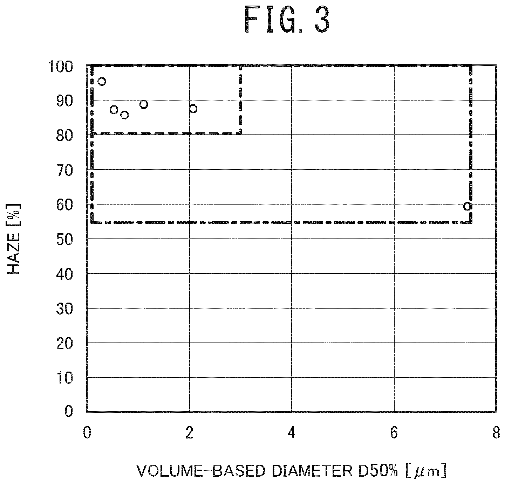

These results are illustrated in Table 1 together with the constitutions of the powders. The relation between total light transmittances and hazes of the powders is illustrated as a graph in FIG. 1. The relation between volume-based diameters D50% and total light transmittances of the titanium phosphate powders prepared in Examples is illustrated in FIG. 2, and the relation between volume-based diameters D50% and hazes is illustrated in FIG. 3.

**[Table 1]**

| No. | Constitution | | | | Performance | | |
|---|---|---|---|---|---|---|---|
| | Material | Volume-based diameter D50% [µm] | Volume-based thickness D50% [µm] | Aspect ratio | Total light transmittance | Haze | Wrinkle blurring effect |
| 1 | Titanium phosphate | 0.29 | 0.030 | 10 | 89.5 | 95.3 | High |
| 2 | Titanium phosphate | 0.53 | 0.065 | 8 | 85.1 | 87.2 | High |
| 3 | Titanium phosphate | 0.74 | 0.090 | 8 | 85.4 | 85.7 | Very high |
| 4 | Titanium phosphate | 1.11 | 0.143 | 8 | 87.1 | 88.7 | Very high |
| 5 | Titanium phosphate | 2.07 | 0.302 | 7 | 90.3 | 87.5 | High |
| 6 | Titanium phosphate | 7.44 | 0.856 | 9 | 91.4 | 59.3 | Somewhat high |
| 7 | Titanium dioxide | 0.29 | Not plate-shaped crystals | | 56.4 | 94.6 | Low |
| 8 | Boron nitride | 4.24 | 0.395 | 11 | 74.6 | 97.9 | Low |
| 9 | Boron nitride | 9.75 | 0.966 | 10 | 84.8 | 79.0 | Somewhat low |
| 10 | Boron nitride | 9.94 | 2.088 | 5 | 88.7 | 50.8 | Somewhat low |

From the results, the followings are found.

The titanium phosphate powders in No. 1 to No. 6 as powders including plate-shaped crystal particles of titanium phosphate, in which the plate-shaped crystal particles had a volume-based diameter D50% of 0.1 um or more and 7.5 um or less and an aspect ratio of 5 or more, had a total light transmittance of 85.1% or more and 91.4% or less and a haze of 59.3% or more and 95.3% or less. In other words, the powders satisfied both a total light transmittance of 85.0% or more and a haze of 59.0% or more.

Of them, the titanium phosphate powders in No. 1 to No. 5, in which the plate-shaped crystal particles had a volume-based diameter D50% of 0.1 um or more and 3.0 um or less and an aspect ratio of 5 or more, had a total light transmittance of 85.1% or more and 90.3% or less and a haze of 85.7% or more and 95.3% or less. In other words, the powders satisfied both a total light transmittance of 85.0% or more and a haze of 85.0% or more.

In contrast, the titanium dioxide powder in No. 7 had a high haze of 94.6% but had a low total light transmittance of 56.4% (60% or less). The boron nitride powders in No. 8 to No. 10 had a total light transmittance or haze of less than 80%, and the boron nitride powder in No. 10 had a particularly low haze of 50.8%.

The wrinkle blurring effect is considered as follows.

The titanium phosphate powders in No. 1 to No. 5, in which the plate-shaped crystal particles had a volume-based diameter D50% of 0.1 um or more and 3.0 um or less and an aspect ratio of 5 or more, satisfied both a total light transmittance of 85.0% or more and a haze of 85.0% or more and thus had high transparency and high wrinkle blurring effect. Of them, the powders in No. 2 and No. 3 had markedly high wrinkle blurring effect. In other words, the titanium phosphate powders in No. 1 to No. 5 had excellent characteristics as a soft focus filler.

The titanium phosphate powder in No. 6, in which the plate-shaped crystal particles had a volume-based diameter D50% of 7.44 um and an aspect ratio of 9, had a high total light transmittance of 91.4%, which indicated high transparency, but had a haze of 59.3%, and had somewhat high wrinkle blurring effect. In other words, the titanium phosphate powder in No. 6 was usable as a soft focus filler.

The titanium dioxide powder in No. 7, which was not plate-shaped crystals and had a volume-based diameter D50% of 0.29 um, had a high haze of 94.6% but had a low total light transmittance of 56.4%. The powder thus got into wrinkles of an artificial leather, and this made the wrinkles stand out. The powder was thus evaluated to have low wrinkle blurring effect. In other words, the titanium dioxide powder in No. 7 is thought to be unsuitable for use as a soft focus filler.

The boron nitride powder in No. 8, which had a volume-based diameter D50% of 4.24 um and an aspect ratio of 11, had a high haze of 97.9% but had a somewhat low total light transmittance of 74.6%. The powder thus got into wrinkles of an artificial leather, and this made the wrinkles stand out. The powder was thus evaluated to have low wrinkle blurring effect. In other words, the boron nitride powder in No. 8 is thought to be unsuitable for use as a soft focus filler.

The boron nitride powders in No. 9 and No. 10 had a high total light transmittance of 84.8% and 88.7%, which indicated high transparency, and had a high volume-based diameter D50% of 9.75 um and 9.94 um, respectively. The powders thus did not get into wrinkles of an artificial leather. The powders, however, had a low haze of 79.0% and 50.8% and were evaluated to have somewhat low wrinkle blurring effect. In other words, the boron nitride powders in No. 9 and No. 10 are thought to be unsuitable for use as a soft focus filler.

### [Preparation of cosmetic composition]

A cosmetic composition containing each powder in No. 1 to No. 10 as the white pigment was prepared. The titanium phosphate powders in No. 1 to No. 5, the titanium dioxide powder in No. 7, and the boron nitride powder in No. 8, each having a haze of 80% or more, achieved high soft focus effect. Of them, the titanium dioxide powder in No. 7 and the boron nitride powder in No. 8 had a total light transmittance of less than 80% and thus achieved poor transparency.

The titanium phosphate powder in No. 6 had a haze of not more than 60%, and thus the soft focus effect was not so high. The powder, however, had a total light transmittance of more than 91%, and thus the transparency was high.

In the titanium phosphate powders in No. 1 to No. 6, the crystals had a plate-shaped shape (had an aspect ratio of 5 or more), and thus the prepared cosmetic composition also had excellent slidability.

## Claims

1. A soft focus filler comprising:
a powder including plate-shaped crystal particles of titanium phosphate.

2. The soft focus filler according to claim 1, wherein the plate-shaped crystal particles have a volume-based 50% cumulative primary particle diameter (volume-based diameter D50%) of 0.1 um or more and 7.5 um or less, and
the plate-shaped crystal particles have an aspect ratio of 5 or more, where the aspect ratio is calculated by dividing the volume-based 50% cumulative primary particle diameter by a volume-based 50% cumulative thickness (volume-based thickness D50%).

3. The soft focus filler according to claim 1, wherein the plate-shaped crystal particles have a volume-based 50% cumulative primary particle diameter (volume-based diameter D50%) of 0.1 um or more and 3.0 um or less, and
the plate-shaped crystal particles have an aspect ratio of 5 or more, where the aspect ratio is calculated by dividing the volume-based 50% cumulative primary particle diameter by a volume-based 50% cumulative thickness (volume-based thickness D50%).

4. The soft focus filler according to claim 1, wherein the soft focus filler has a total light transmittance of 80% or more and a haze of 80% or more.

5. The soft focus filler according to any one of claims 1 to 4, wherein the plate-shaped crystal particles are hexagonal plate-shaped crystal particles.

6. A cosmetic comprising the soft focus filler according to any one of claims 1 to 5.
